# EUROPEAN PATENT APPLICATION

(11) **EP 2 915 805 A1**
(43) Date of publication of application: **09.09.2015**
(21) Application number: 13850807.2
(22) Date of filing: 25.10.2013
(51) Int. Cl.: C07D 211/60, C07D 471/08, C07D 487/08, C07B 53/00, C07B 61/00

(54) **METHOD FOR PRODUCING OPTICALLY ACTIVE BICYCLIC UREA COMPOUND**

(30) Priority: 01.11.2012 JP 2012242227; 29.01.2013 JP 2013014760
(71) Applicant: Kaneka Corporation, Osaka-shi, Osaka 530-8288 (JP)
(72) Inventor: HIRAI, Yoshinori, Takasago-shi Hyogo 676-8688 (JP); NISHIYAMA, Akira, Takasago-shi Hyogo 676-8688 (JP)
(74) Representative: Hoffmann Eitle
(86) International application number: PCT/JP2013/078918
(87) International publication number: WO 2014/069351

(57) **Abstract**

A problem to be solved by the present invention is to provide a process for producing an optically active bicyclic urea compound useful as an intermediate for β-lactamase inhibitor, in a simple and easy manner with high efficiency.

The present invention includes reacting a specific ester compound with a specific amine in the presence of a metal alkoxide and/or an alkaline earth metal salt to produce the corresponding amide compound, which is then reacted with phosgene or a phosgene equivalent, followed by, if necessary, treatment with an acid or a base, to produce an optically active bicyclic urea compound. This makes it possible to produce an optically active bicyclic urea compound in a simple and easy manner with high efficiency and in high optical purity, without using expensive reagents such as catalysts and condensation agents, and without passing through protection and deprotection steps.

## Description

### TECHNICAL FIELD

The present invention relates to a process for producing an optically active bicyclic urea compound useful as an intermediate for medicines, particularly as an intermediate for β-lactamase inhibitor.

### BACKGROUND ART

As a process for producing an optically active bicyclic urea compound to be used as an intermediate for β-lactamase inhibitor, there has been known a process as shown below (see Patent Document 1, particularly PREPARATIVE EXAMPLE 1, Example 1A).

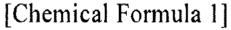

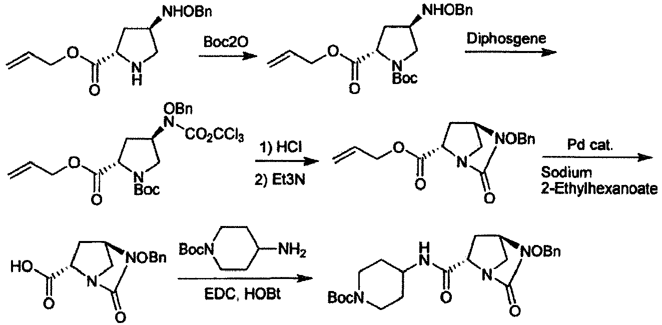

### PRIOR ART DOCUMENTS

### PATENT DOCUMENTS

Patent Document 1: WO 2009/091856

### SUMMARY OF THE INVENTION

### PROBLEM TO BE SOLVED BY THE INVENTION

The process disclosed in Patent Document 1 has a problem in the practice on an industrial scale in terms of including many steps and using expensive reagents such as palladium and dehydration-condensation agents.

### MEANS FOR SOLVING THE PROBLEMS

Under the above circumstances, the present inventors have earnestly studied, and as a result, they have found that an optically active bicyclic urea compound can be obtained by reacting a specific ester compound with a specific amine in the presence of a metal alkoxide and/or an alkaline earth metal salt to produce the corresponding amide compound and further reacting the amide compound with phosgene or a phosgene equivalent, followed by treatment with an acid, thereby completing the present invention.

That is, the present invention relates to a process for producing an amide compound of formula (4):

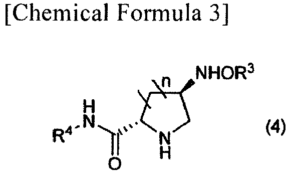

wherein R³, R⁴, and n are the same as defined below, comprising:
reacting an ester compound of formula (2): 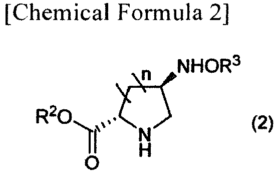 wherein R² and R³ are each independently C₁₋₁₂ alkyl group, C₂₋₁₂ alkenyl group, C₃₋₁₂ cycloalkyl group, C₇₋₁₂ aralkyl group, or C₆₋₁₂ aryl group; and n is 1 or 2, with an amine of formula (3):
R⁴NH₂ (3)

wherein R⁴ is hydrogen atom, C₁₋₁₂ alkyl group, C₂₋₁₂ alkenyl group, C₃₋₁₂ cycloalkyl group, C₇₋₁₂ aralkyl group, or C₆₋₁₂ aryl group, in the presence of a metal alkoxide of formula (1):

M(OR¹)_{X} (1)

wherein M is magnesium, aluminum, titanium, zirconium, or hafnium; R¹ is C₁₋₁₂ alkyl group; and X is an integer of 1 to 4, and/or an alkaline earth metal salt.

The above amide compound can be converted into an optically active bicyclic urea compound of formula (5):

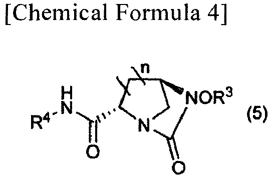

by reacting the compound (4) with phosgene or a phosgene equivalent.

### EFFECTS OF THE INVENTION

The process of the present invention makes it possible to produce an optically active bicyclic urea compound useful as an intermediate for medicines, particularly as an intermediate for β-lactamase inhibitor, in a simple and easy manner without using expensive reagents such as catalysts and condensation agents, and without passing through protection and deprotection steps, as well as in high optical purity without causing racemization.

### MODE FOR CARRYING OUT THE INVENTION

First, the following will describe the starting materials to be used in the present invention.

The ester compound to be used in the present invention is of formula (2): 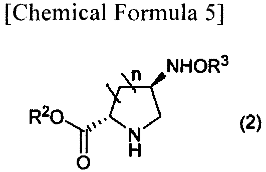 wherein R² and R³ are each independently C₁₋₁₂ alkyl group, C₂₋₁₂ alkenyl group, C₃₋₁₂ cycloalkyl group, C₇₋₁₂ aralkyl group, or C₆₋₁₂ aryl group.

These groups may have at least one substituent group. Examples of the substituent group may include halogen atoms such as fluorine atom, chlorine atom, bromine atom, and iodine atom; hydroxy group; alkoxy groups such as methoxy group and ethoxy group; methylthio group; trifluoromethyl group; acetyl group; benzoyl group; cyano group; nitro group; carboxyl group; and alkoxycarboxyl groups such as methoxycarboxyl group and ethoxycarboxyl group.

Examples of the alkyl group may include straight chain or branched alkyl groups such as methyl group, ethyl group, n-propyl group, isopropyl group, n-butyl group, isobutyl group, sec-butyl group, tert-butyl group, n-pentyl group, hexyl group, heptyl group, octyl group, nonyl group, decyl group, undecyl group, and dodecyl group. The carbon number of the alkyl group may preferably be 1 to 7, more preferably 1 to 5, and particularly preferably 1 to 3.

Examples of the alkenyl group may include vinyl group, allyl group, 1-propenyl group, isopropenyl group, 3-butenyl group, and 1-methylallyl group. In particular, preferred are C₂₋₅ alkenyl groups, with more preferred being C₂₋₄ alkenyl groups and still more preferred being allyl group.

The cycloalkyl group is defined as a group derived from cycloalkane by removing one hydrogen atom from the cycloalkane, of which at least one carbon atom, preferably one carbon atom, may be substituted with at least one heteroatom selected from nitrogen atom, oxygen atom, and sulfur atom. Therefore, the carbon number of the cycloalkyl group exactly means the total number of carbon atoms and heteroatoms, and it corresponds to the number of elements forming the ring skeleton, e.g., it means a three-membered ring in the case of C₃. Specific examples of the C₃₋₁₂ cycloalkyl group may include cyclic saturated hydrocarbon groups such as cyclopropyl group, cyclobutyl group, cyclopentyl group, cyclohexyl group, cycloheptyl group, cyclooctyl group, and cyclododecyl group; nitrogen-containing saturated heterocyclic groups such as azirin-2-yl group, azetidin-2-yl group, azetidin-3-yl group, pyrrolidin-2-yl group, pyrrolidin-3-yl group, piperidin-2-yl group, piperidin-3-yl group, piperidin-4-yl group, azepan-2-yl group, azepan-3-yl group, and azepan-4-yl group; oxygen-containing saturated heterocyclic groups such as oxiran-2-yl group, oxetan-2-yl group, oxetan-3-yl group, oxolan-2-yl group, oxolan-3-yl group, oxan-2-yl group, oxan-3-yl group, oxan-4-yl group, oxepan-2-yl group, oxepan-3-yl group, and oxepan-4-yl group; and sulfur-containing saturated heterocyclic groups such as thiiran-2-yl group, thietan-2-yl group, thietan-3-yl group, thiolan-2-yl group, thiolan-3-yl group, thian-2-yl group, thian-3-yl group, thian-4-yl group, thiepan-2-yl group, thiepan-3-yl group, and thiepan-4-yl group. The number of elements forming the ring skeleton of the cycloalkyl group is 3 to 12, preferably 5 to 7, and more preferably 6, i.e., six-membered ring.

When the cycloalkyl group is a nitrogen-containing saturated heterocyclic group, the nitrogen atom of the heterocyclic group may be protected with a protecting group. Examples of the protecting group on the nitrogen atom may include C₁₋₁₅ substituted or unsubstituted alkyloxycarbonyl groups such as t-butyloxycarbonyl group (Boc group), methoxycarbonyl group (Moc group), and 9-fluorenylmethoxycarbonyl group (Fmoc group); C₇₋₁₂ substituted or unsubstituted aralkyloxycarbonyl groups such as benzyloxycarbonyl group (Cbz group) and p-methoxybenzyloxycarbonyl group; and C₂₋₁₂ substituted or unsubstituted acyl groups such as acetyl group and benzoyl group. Preferred are t-butyloxycabonyl group (Boc group), methoxycarbonyl group (Moc group), 9-fluorenylmethoxycarbonyl group (Fmoc group), benzyloxycarbonyl group (Cbz group), and acetyl group, with more preferred being t-butyloxycarbonyl group (Boc group) and benzyloxycarbonyl group (Cbz group).

Examples of the aralkyl group may include benzyl group, 1-phentyl group, 2-phenetyl group, phenylpropyl group, phenylbutyl group, and phenylpentyl group. Preferred is C₇₋₁₀ aralkyl group, with more preferred being C₇₋₈ aralkyl group.

Examples of the aryl group may include phenyl group, tolyl group, xylyl group, mesytyl group, duryl group, and 1-naphtyl group.

Specific preferred examples of the R² or R³ may include C₁₋₈ alkyl groups, preferably C₁₋₅ alkyl groups, such as methyl group, ethyl group, n-propyl group, isopropyl group, n-butyl group, isobutyl group, sec-butyl group, tert-butyl group, and n-pentyl group; C₃₋₇ cycloalkyl groups, preferably C₃₋₅ cycloalkyl groups, such as cyclopropyl group, cyclobutyl group, and cyclohexyl group; alkenyl groups such as allyl group; C₇₋₁₂ aralkyl groups, preferably C₇₋₉ aralkyl groups, such as benzyl group and 1-phenetyl group; and aryl groups such as phenyl group and 1-naphtyl group.

More preferred as the R² is C₁₋₃ alkyl group such as methyl group, ethyl group, or isopropyl group; aryl group, or benzyl group, with still more preferred being benzyl group.

More preferred as the R³ is allyl group or benzyl group, with still more preferred being benzyl group.

The n is 1 or 2, with preferred being a piperidine derivative when n is 2.

The compound (2) can easily be produced in accordance with the method disclosed in WO2002/010172 or CHEMICAL COMMUNICATIONS, 1996, 349. More specifically, it can be produced by the method described below. Still more specifically, the compound (2) can be produced as follows: a glutamic acid derivative is reacted with diazomethane or the like to give the corresponding diazoketone, which is then subjected to intermolecular cyclization using a rhodium catalyst or the like. The carbonyl group is then reduced with a reducing agent such as sodium borohydride, followed by deprotection of the protecting group on the nitrogen atom, to produce the compound (2). The deprotection can be performed by conversion of, for example, tert-butoxycarbonyl group as a protecting group into trifluoroacetyl group and triflation of the resultant alcohol compound, followed by substitution reaction with benzyloxyamine.

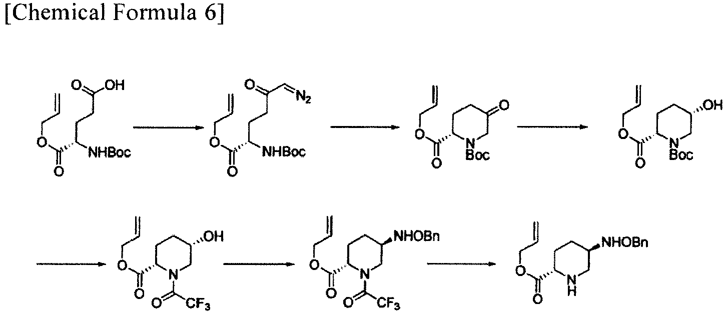

The following will describe the amide compound as a product.

The amide compound obtained in the present invention is of formula (4): 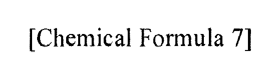 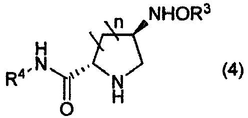 wherein R³ and n are the same as defined above.

R⁴ is hydrogen atom, C₁₋₁₂ alkyl group, C₂₋₁₂ alkenyl group, C₃₋₁₂ cycloalkyl group, C₇₋₁₂ aralkyl group, or C₆₋₁₂ aryl group. These may have at least one substituent group. Examples of the substituent and respective functional groups may include the same groups as described above for the case of R² and R³.

Preferred examples of R⁴ may include C₁₋₅ alkyl groups each optionally having at least one substituent group; C₂₋₄ alkenyl groups; C₃₋₆ cyclic saturated hydrocarbon groups each optionally having at least one substituent group; C₃₋₆ nitrogen-containing saturated heterocyclic groups each optionally having at least one substituent group and optionally being protected on at least one nitrogen atom; C₇₋₈ aralkyl groups each optionally having at least one substituent group; and C₆₋₁₂ aryl groups each optionally having at least one substituent group.

Specific examples of the R⁴ may include hydrogen atom, methyl group, ethyl group, n-propyl group, isopropyl group, cyclopropyl group, n-butyl group, isobutyl group, sec-butyl group, tert-butyl group, cyclobutyl group, n-pentyl group, cyclohexyl group, allyl group, benzyl group, 1-phenetyl group, phenyl group, 1-naphtyl group, p-methoxyphenyl group, 1-(tert-butoxycarbonyl)-piuperidin-4-yl group, and 1-(benzyloxycarbonyl)piperidin-4-yl group.

Particularly preferred as the R⁴ is hydrogen atom, allyl group, benzyl group, p-methoxyphenyl group, 1-(tert-butoxycarbonyl)piperidin-4-yl group, or 1-(benzyl-oxycarbonyl)piperidin-4-yl group, with more particularly preferred being hydrogen atom or 1-(benzyl-oxycarbonyl)piperidin-4-yl group.

The following will describe an optically active bicyclic urea compound to be obtained by reaction of the amide compound with phosgene or a phosgene equivalent.

The optically active bicyclic urea compound to be obtained in the present invention is of formula (5): 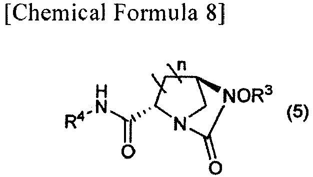 wherein R³, R⁴, and n are the same as defined above.

The following will describe a process for producing the compound (4).

The compound (4) can be produced by reacting the compound (2) with an amine of formula (3):

R⁴NH₂ (3)

in the presence of a metal alkoxide of formula (1):

M(OR¹)_{X} (1)

and/or an alkaline earth metal salt.

In the metal alkoxide (1), M is a metal selected from magnesium, aluminum, titanium, zirconium, or hafnium, preferably magnesium, aluminum, or titanium, more preferably aluminum or titanium. The use of the metal alkoxide (1) wherein the M is aluminum or titanium makes it possible to perform the above reaction in high yield not lower than 80%. In particular, the metal alkoxide (1) wherein the M is aluminum is preferred because it can shorten the reaction time significantly down to 10 hours or shorter, thereby enabling the reaction to be caused with high efficiency.

The R¹ represents a C₁₋₁₂ alkyl group. Specific examples of the alkyl group may include methyl group, ethyl group, n-propyl group, isopropyl group, n-butyl group, isobutyl group, sec-butyl group, tert-butyl group, and n-pentyl group. Preferred are C₁₋₅ alkyl groups, with more preferred being methyl group, ethyl group, isopropyl group, and tert-butyl group. The R¹ may preferably is in a branched form, more preferably isopropyl group.

The X is an integer of 1 to 4, and is determined by the kind of metal.

The metal alkoxide (1) may preferably be Ti(OMe)4, Ti(OEt)₄, Ti(O-i-Pr)₄, Al(OMe)₃, Al(OEt)₃, Al(O-i-Pr)₃, Mg(OMe)₂, Mg(OEt)₂, Hf(O-t-Bu)₄, or Zr(O-n-Bu)₄, more preferably Ti(OMe)₄, Ti(OEt)₄, Ti(O-i-Pr)₄, Al(OMe)3, Al(OEt)₃, Al(O-i-Pr)₃, Mg(OMe)₂, or Mg(OEt)₂, and more preferably Ti(O-i-Pr)₄ or Al(O-i-Pr)₃.

In this step, an alkaline earth metal salt can also be used as a catalyst in place of, or in addition to, the metal alkoxide (1). The "alkaline earth metal salt" as used herein may include alkaline earth metal halides and alkaline earth metal oxides, of which examples may include calcium chloride, calcium oxide, calcium bromide, magnesium chloride, magnesium bromide and magnesium oxide, with preferred being calcium chloride and magnesium chloride, particularly preferably calcium chloride. When the compound (4) as a product is to be obtained in high yield, magnesium chloride may preferably be used.

The amount of the metal alkoxide (1) or alkaline earth metal salt to be used is not smaller than 0.1 mole, preferably not smaller than 0.5 mole, and more preferably not smaller than 1.0 mole, relative to 1 mole of the compound (2), as the lower limit, and is not greater than 10 moles, preferably not greater than 5 moles, and not greater than 2 moles, relative to 1 mole of the compound (2), as the upper limit.

In the amine (3), the R⁴ is the same as defined above. Preferred examples of the amine (3) may include ammonia, allylamine, benzylamine, p-methoxyaniline, 4-amino-1-(tert-butoxycarboxyl)-piperidine, and 4-amino-1-(benzyloxycarbonyl)-piperidine, with more preferred being ammonia or 4-amino-1-(benzyloxycarbonyl)-piperidine.

The amount of the amine (3) to be used is not smaller than 0.5 mole, preferably not smaller than 0.8 mole, and more preferably not smaller than 1 mole, relative to 1 mole of the compound (2), as the lower limit, and is not greater than 50 moles, preferably not greater than 20 moles, and more preferably not greater than 10 moles, relative to 1 mole, relative to 1 mole of the compound (2), as the upper limit.

The reaction solvent to be used in this step is not particularly limited, so long as it has no adverse effect on the reaction. For example, an ordinary solvent such as a hydrocarbon solvent, an ether solvent, or a halogen solvent is used as the reaction solvent. Specific examples of the reaction solvent may include hydrocarbon solvents such as pentane, hexane, heptane, cyclopentane, cyclohexane, cycloheptane, benzene, toluene, xylene, and mesitylene; ether solvents such as diethyl ether, diisopropyl ether, cyclopentyl methyl ether, tert-butyl methyl ether, tetrahydrofuran, 1,4-dioxane, and ethylene glycol dimethyl ether; and halogen solvents such as methylene chloride, 1,2-dichloroethane, 1,1-dichloroethane, and tetrachloroethane, with preferred being aromatic hydrocarbon solvents such as benzene, toluene, xylene, and mesitylene; dialkyl ethers such as diethyl ether, diisopropyl ether, cyclopentyl methyl ether, and tert-butyl methyl ether; and cyclic ethers such as tetrahydrofuran, 1, 4-dioxane, and ethylene glycol dimethyl ether, more preferably toluene and tetrahydrofuran.

The amount of the reaction solvent to be used may preferably be not greater than 50 parts by weight, more preferably not greater than 20 parts by weight, relative to 1 part by weight of the compound (2), as the upper limit, because too great amounts are not preferred in terms of cost or effort for post-treatment. On the other hand, the lower limit thereof is not particularly limited, but may preferably be not smaller than 0.5 part by weight, more preferably not smaller than 1 part by weight, relative to 1 part by weight of the compound (2).

The reaction temperature to be used in this step is not particularly limited, so long as it is not lower than the freezing point and not higher than the boiling point of the solvent, but it may appropriately be determined, with the preferred lower limit thereof being not lower than -78°C, more preferably not lower than -50°C, still more preferably not lower than -30°C, and particularly preferably not lower than 15°C, and the preferred upper limit thereof being not higher than 150°C, more preferably not higher than 100°C, and particularly preferably not higher than 50°C.

The reaction time to be used in this step is not shorter than 0.5 hour and not longer than 100 hours, preferably not longer than 50 hours. The use of the metal alkoxide (1) wherein the M is aluminum makes it possible to shorten the reaction time down to 10 hours or shorter with expectancy.

The order of the addition of reagents in this step is not particularly limited, but a metal alkoxide and/or an alkaline earth metal salt, as well as the compound (2), may successively be added to a solution of the compound (3); the compound (3) and the compound (2) may be added to a solution of a metal alkoxide and/or an alkaline earth metal salt; or the compound (3), as well as a metal alkoxide and/or an alkaline earth metal salt, may be added to a solution of the compound (2). In the addition of a metal alkoxide and/or an alkaline earth metal salt, as well as the compounds (2) and (3), these reagents may be added after dissolved in an appropriate solvent.

After the reaction in this step, ordinary treatment may be performed as the post-treatment to obtain a product from the reaction solution. More specifically, for example, water is added to the reaction solution, followed by extraction with addition of an organic solvent such as toluene, ethyl acetate, isopropyl acetate, methyl tert-butyl ether, or hexane. The organic solvent is distilled away from the resultant extract by an operation such as heating under reduced pressure, whereby a target product can be isolated. In the case where insoluble matter is precipitated, the insoluble matter may be filtered away, followed by an operation as described above. The target product thus obtained has purity enough to be usable in the subsequent step, but it may further be purified by any of ordinary purification methods such as crystallization, fractional distillation, and column chromatography.

The thus obtained compound of formula (6) wherein R³ is Bn, R⁴ is H, and n is 2 is a compound unknown to the public through publication.

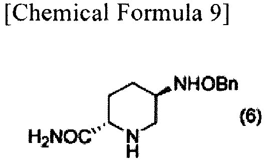

The following will describe a process for producing the compound (5).

The compound (5) is produced by reacting the compound (4) with phosgene or a phosgene equivalent.

Examples of the phosgene equivalent may include diphosgene, triphosgene, and carbonyldiimidazole.

The amount of phosgene or phosgene equivalent to be used is not smaller than 0.3 mole, preferably not smaller than 0.5 mole, and more preferably not smaller than 0.8 mole, relative to 1 mole of the compound (4), as the lower limit, and is not greater than 10 moles, preferably not greater than 5 moles, and not greater than 3 moles, relative to 1 mole of the compound (4), as the upper limit.

The reaction solvent to be used in this step is not particularly limited, so long as it has no adverse effect on the reaction. For example, ordinary solvents such as hydrocarbon solvents, ether solvents, ester solvents, halogen solvents and nitrile solvents may be used as the reaction solvent. Specific examples of the reaction solvent may include hydrocarbon solvents such as pentane, hexane, heptane, cyclopentane, cyclohexane, cycloheptane, benzene, toluene, xylene, and mesitylene; ether solvents such as diethyl ether, diisopropyl ether, cyclopentyl methyl ether, tert-butyl methyl ether, tetrahydrofuran, 1,4-dioxane, and ethylene glycol dimethyl ether; ester solvents such as methyl acetate, ethyl acetate, isopropyl acetate, and butyl acetate; halogen solvents such as methylene chloride, 1,2-dichloroethane, 1,1-dichloroethane, and tetrachloroethane; and nitrile solvents such as acetonitrile, with preferred being toluene, tetrahydrofuran, methylene chloride, and acetonitrile, more preferably toluene, tetrahydrofuran, methylene chloride, and acetonitrile.

The reaction temperature in this step is not particularly limited, so long as it is not lower than the freezing point and not higher than the boiling point of the solvent, but it may appropriately be determined, with the preferred lower limit thereof being not lower than -78°C, more preferably not lower than -50°C, and particularly preferably not lower than -30°C, and the preferred upper limit thereof being not higher than 150°C, more preferably not higher than 100°C, still more preferably not higher than 50°C, and particularly preferably not higher than 20°C.

The reaction time in this step is preferably not shorter than 0.1 hour, more preferably not shorter than 0.5 hour, and still more preferably not shorter than 0.7 hour, and is preferably not longer than 50 hours, more preferably not longer than 20 hours, still more preferably not longer than 10 hours, and particularly preferably not longer than 5 hours.

The order of the addition of reagents in this step is, for example, as follows: phosgene or a phosgene equivalent may be added to a solution of the compound (4), or alternatively, the compound (4) may be added to a solution of phosgene or a phosgene equivalent.

The reaction of the compound (4) with phosgene or a phosgene equivalent may produce a compound of formula (7): 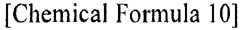 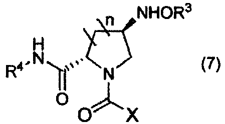 or a compound of formula (8): 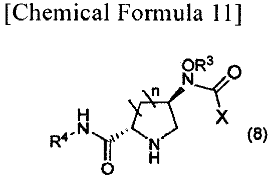 wherein X is the residue of phosgene or a phosgene equivalent. In such a case, the compound (5) can be obtained by treating the reaction solution with an acid or a base.

Examples of the acid may include hydrochloric acid, hydrobromic acid, sulfuric acid, phosphoric acid, acetic acid, and citric acid. These may preferably be used as an aqueous solution. Preferred is phosphoric acid.

The amount of the acid to be used is not smaller than 0.3 mole, preferably not smaller than 0.5 mole, more preferably not smaller than 0.8 mole, relative to 1 mole of the compound (4), as the lower limit, and is not greater than 10 moles, preferably not greater than 5 moles, and more preferably not greater than 3 moles, relative to 1 mole of the compound (4), as the upper limit.

Examples of the base may include pyridine, triethylamine, diisopropylethylamine, N,N-dimethylaminopyridine, sodium hydrogencarbonate, potassium hydrogencarbonate, sodium carbonate, and potassium carbonate, with preferred being N,N-dimethylaminopyridine. The amount of the base to be used is not smaller than 0.3 mole, preferably not smaller than 0.5 mole, and more preferably not smaller than 0.8 mole, relative to 1 mole of the compound (4), as the lower limit, and is not greater than 10 moles, preferably not greater than 5 moles, and more preferably not greater than 3 moles, relative to 1 mole of the compound (4), as the upper limit.

The acid or base may be added when the reaction of the compound (4) with phosgene or a phosgene equivalent has proceeded to a certain extent. Alternatively, phosgene or a phosgene equivalent may be added to a solution of the compound (4) and a base.

After the reaction in this step, ordinary treatment may be performed as the post-treatment to obtain a product from the reaction solution. More specifically, for example, water is added to the reaction solution, followed by extraction with addition of an organic solvent such as toluene, ethyl acetate, isopropyl acetate, methyl tert-butyl ether, or hexane. The organic solvent is distilled away from the resultant extract by an operation such as heating under reduced pressure, whereby a target product can be isolated.

The target product thus obtained may be subjected to any of ordinary purification methods such as crystallization, fractional distillation, and column chromatography.

The present application claims the benefit of priority based on Japanese Patent Application No. 2012-242227, filed on November 1, 2012, and Japanese Patent Application No. 2013-014760, filed on January 29, 2013. The specifications of Japanese Patent Application No. 2012-242227, filed on November 1, 2012, and Japanese Patent Application No. 2013-014760, filed on January 29, 2013 are incorporated herein in their entirety by reference.

### EXAMPLES

The present invention will be described below in more detail by way of Examples, but these Examples are not to be construed as any limitation to the present invention.

In the Examples described below, the respective compounds were analyzed for their yields and production ratios by high-performance liquid chromatography under the following conditions.

High-performance Liquid Chromatography Analysis Conditions
Column: Phenomenex Luna 5C18(2), 4.6 x250 mm
Mobile phase A: 0.1 wt% aqueous phosphoric acid solution
Mobile phase B: acetonitrile
Flow rate: 1.0 mL/min
Gradient condition:
Mobile phase A : B = 80 : 20 at 0.00 minutes
Mobile phase A : B = 20 : 80 at 30.00 minutes
Mobile phase A : B = 20 : 80 at 40.00 minutes
Mobile phase A : B = 80 : 20 at 40.01 minutes
Stop at 53.00 minutes
Column temperature: 35°C
Detection wavelength: 210 nm

### Example 1: Production process for 4-[({(2S,5R)-5-[(benzyloxy)amino]-piperidin-2-yl}carbonyl)amino]piperidine-1-carboxylic acid benzyl ester

4-Amino-1-(benzyloxycarbonyl)piperidine (6.31 g, 26.9 mmol) and Ti(O-i-Pr)4 (7.61 g, 26.8 mmol) were added to tetrahydrofuran (30 mL), followed by stirring at 25°C for 1 hour. To the reaction solution was added (2S,5R)-5-(benzyloxyamino)-piperidine-2-carboxyolic acid benzyl ester (7.57 g, 22.3 mmol), followed by stirring at 35°C for 23 hours. Water (38 mL) and saturated aqueous sodium hydrogencarbonate solution (20 mL) were added in order, and the precipitated insoluble matter was filtered away. The filtrate was removed from aqueous layer, and the organic layer was washed with water (20 mL). The organic layer was concentrated to 31 g under reduced pressure, and hexane (50 mL) was added at 50°C, followed by cooling to 0°C. After stirring for 1 hour, precipitated solid was filtered out and then washed with hexane (40 mL), followed by drying at 40°C under reduced pressure, to obtain the title compound (8.38 g, 81% yield).

### Example 2: Production process for 4-({[(2S,5R)-6-(benzyloxy)-7-oxo-1,6-diazabicyclo[3.2.1]octan-2-yl]carbonyl}amino)piperidine-1-carboxylic acid benzyl ester

To the compound (8.07 g, 17.3 mmol) obtained in Example 1 were added diisopropylethylamine (7.19 g, 55.7 mmol) and methylene chloride (144 mL), followed by cooling at -10°C. To the reaction solution was added triphosgene (4.11 g, 13.9 mmol), followed by stirring at 25°C for 1 hour. To the reaction solution was added 3 wt% aqueous phosphoric acid solution (45 g), followed by stirring at 25°C for 21 hours and removing aqueous layer. The organic layer was washed successively with 5 wt% aqueous sodium hydrogencarbonate solution (40mL) and water (40 mL) in order. The organic layer was concentrated under reduced pressure to obtain the title compound (8.25 g, 97% yield).

### Example 3: Production process for (2S,5R)-5-(benzyloxyamino)-piperidine-2-carboxylic acid amide

To (2S,5R)-5-(benzyloxyamino) -piperidine-2-carboxylic acid ethyl ester (278 mg, 1 mmol) were added calcium chloride (111 mg, 1.00 mmol) and 20 wt% ammonia methanol solution (860 mg), followed by stirring at 25 °C for 4 hours. The quantitative determination of the reaction solution by high-performance liquid chromatography revealed that the title compound had been produced in 86% yield. The reaction solution was concentrated under reduced pressure, after which ethyl acetate (20 mL) and water (2 mL) were added, and the precipitated product was filtered out. The precipitated product was washed with ethyl acetate (2 mL), followed by drying under reduced pressure, to obtain the title compound (154 mg, 62% yield).
¹H NMR (500 MHz, CDCl₃): 7.27-7.35 (m, 5H), 4.65 (s, 2H), 3.74 (dd, 1H, 12.0, 3.5Hz), 3.51 (ddd, 1H, 12.0, 4.0, 1.5Hz), 3.15-3.21 (m, 1H), 2.78 (t, 1H, 12.0Hz), 2.30 (dq, 1H, 14.0Hz, 3.5Hz), 1.98-2.03 (m, 1H), 1.75 (dq, 1H, 12.0Hz, 3.5Hz).

### Example 4: Production process for (2S,5R)-5-(benzyloxyamino)-piperidine-2-carboxylic acid amide

The same production was performed as described in Example 3, except that magnesium chloride was used in place of calcium chloride. The analysis of the reaction solution by high-performance liquid chromatography revealed that the title compound had been produced in 99% yield.

### Example 5: Production process for 7-oxo-6-benzyloxy-1,6-diazabicylco-[3.2.1]octane-2-carboxylic acid amide

(2S,5R)-5-(benzyloxyamino)-piperidine-2-carboxylic acid amide (118 mg, 0.475 mmol), potassium carbonate (372 mg, 2.69 mmol), and dichloromethane (10 mL) were cooled to -10°C, followed by addition of triphosgene (117 mg, 0.394 mmol). Stirring at -10°C for 1 hour was followed by addition of N,N-dimethylaminopyridine (2.3 mg, 0.019 mmol) and further stirring at room temperature for 1 day. The reaction solution was concentrated, to which ethyl acetate (10 mL) and water (2 mL) were added, and the resultant mixture was removed from aqueous layer. The organic layer was dried with anhydrous sodium sulfate, followed by concentration under reduced pressure, to obtain the title compound (83.3 mg, 64% yield).
¹H NMR (500 MHz, CDCl₃): 7.34-7.44 (m, 5H), 6.69 (s, 1H), 6.20 (s, 1H), 5.05 (d, J = 11.5 Hz, 1H), 4.91 (d, J = 11.5 Hz, 1H), 3.94 (d, J = 8.5 Hz, 1H), 3.32 (s, 1H), 3.03 (d, J = 11.5 Hz, 1H), 2.78 (d, J = 11.5 Hz, 1H), 2.33 (dd, J = 14.5, 7.0 Hz, 1H), 1.88-2.02 (m, 2H), 1.58-1.64 (m, 1H).

### Example 6: Production process for (2S,5R)-5-(benzyloxyamino)-piperidine-2-carboxylic acid allyl amide

Allyl amine (46.8 mg, 0.820 mmol), Al(O-i-Pr)₃ (156 mg, 0.764 mmol), and tetrahydrofuran (1 mL) were stirred at 60°C for 1 hour, to which (2S,5R)-5-(benzyloxyamio)-piperidine-2-carboxylic acid benzyl ester (171 mg, 0.504 mmol) was then added, and the mixture was stirred for 5 hours, after which the quantitative analysis of the reaction solution by high-performance liquid chromatography revealed that the title compound had been produced in 92% yield.
¹H NMR (500MHz, CDCl₃): 7.28-7.35 (m, 5H), 6.86 (s(br), 1H), 5.82 (ddt, J = 17.0, 10.5, 5.5 Hz, 1H), 5.11-5.19 (m, 2H), 4.68 (s, 2H), 3.87 (tt, J = 5.5, 1.5 Hz, 2H), 3.30 (ddd, J = 11.0, 4.0, 1.5 Hz, 1H), 3.17 (dd, J = 11.0, 3.5 Hz, 1H), 2.96 (tt, J = 10.0, 4.0 Hz, 1H), 2.46 (dd, J = 11.5, 10.0 Hz, 1H), 2.11 (dq, J = 13.0, 4.0 Hz, 1H), 1.88-1.94 (m, 1H), 1.42-1.50 (m, 1H), 1.23-1.31 (m, 1H).

### Example 7: Production process for 4-[({(2S,5R)-5-[(benzyloxy)amino]-piperidine-2-yl}carbonyl)amino]piperidine-1-caboxyolic acid benzyl ester

The reaction was performed for 4 hours under the same conditions as described in Example 1, except that Al(O-i-Pr)₃ was used in place of Ti(O-i-Pr)₄. The analysis by high-performance liquid chromatography revealed that the title compound had been produced in 90% yield.

### Example 8: Production process for 4-[({(2S,5R)-5-[(benzyloxy)amino]-piperidine-2-yl}carbonyl)amino]piperidine-1-caboxyolic acid benzyl ester

The reaction was performed for 27 hours under the same conditions as described in Example 1, except that Zr(O-n-Bu)₄ was used in place of Ti(O-i-Pr)₄. The analysis by high-performance liquid chromatography revealed that the title compound had been produced in 38% yield.

### Example 9: Production process for 4-[({(2S,5R)-5-[(benzyloxy)amino]-piperidine-2-yl}carbonyl)amino]piperidine-1-caboxyolic acid benzyl ester

The reaction was performed for 21 hours under the same conditions as described in Example 1, except that Hf(O-t-Bu)₄ was used in place of Ti(O-i-Pr)₄. The analysis by high-performance liquid chromatography revealed that the title compound had been produced in 51 % yield.

### Comparative Example 1: Production process for (2S,5R)-5-(benzyloxyamino)-piperidine-2-carboxylic acid amide

To (2S,5R)-5-(benzyloxyamino)-piperidine-2-carboxylic acid ethyl ester (278 mg, 1 mmol) was added 20 wt% ammonia solution in methanol (860 mg), and stirring was continued at 45°C for 14 hours. The analysis of the reaction solution by high-performance liquid chromatography revealed that the title compound had been produced in 20% yield.

## Claims

1. A process for producing an amide compound of formula (4): 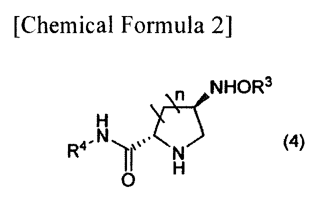 wherein R³, R⁴, and n are the same as defined below, comprising:
reacting an ester compound of formula (2): 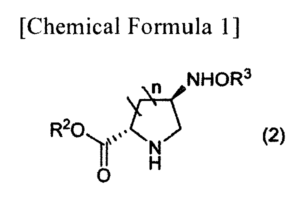 wherein R² and R³ are each independently C₁₋₁₂ alkyl group, C₂₋₁₂ alkenyl group, C₃₋₁₂ cycloalkyl group, C₇₋₁₂ aralkyl group, or C₆₋₁₂ aryl group; and n is 1 or 2, with an amine of formula (3):
R⁴NH₂ (3)
wherein R⁴ is hydrogen atom, C₁₋₁₂ alkyl group, C₂₋₁₂ alkenyl group, C₃₋₁₂ cycloalkyl group, C₇₋₁₂ aralkyl group, or C₆₋₁₂ aryl group, in the presence of a metal alkoxide of formula (1):
M(OR¹)_{X} (1)
wherein M is magnesium, aluminum, titanium, zirconium, or hafnium; R¹ is C₁₋₁₂ alkyl group; and X is an integer of 1 to 4, and/or an alkaline earth metal salt.

2. The production process according to claim 1, wherein the metal alkoxide is Ti(O-i-Pr)₄ or Al(O-i-Pr)₃.

3. The production process according to claim 1, wherein the alkaline earth metal salt is calcium chloride or magnesium chloride.

4. The production process according to any of claims 1 to 3, wherein the R² is methyl group, ethyl group, isopropyl group, allyl group, or benzyl group; R³ is benzyl group; and n is 2.

5. The production process according to any of claims 1 to 4, wherein the R⁴ is hydrogen atom, allyl group, benzyl group, p-methoxyphenyl group, 1-(tert-butoxy-carbonyl)piperidin-4-yl group, or 1-(benzyloxycarbonyl)piperidin-4-yl group.

6. The production process according to any of claims 1 to 5, further comprising reacting the compound (4) with phosgene or a phosgene equivalent to produce an optically active bicyclic urea compound of formula (5): 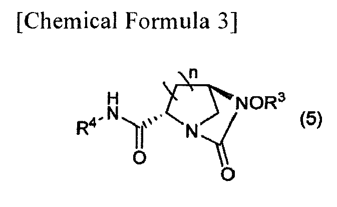 wherein R³, R⁴, and n are the same as defined above.
